# EUROPEAN PATENT SPECIFICATION

(11) **EP 0 486 715 B1**
(45) Date of publication and mention of the grant of the patent: **31.01.1996**
(21) Application number: 90122099.6
(22) Date of filing: 19.11.1990
(51) Int. Cl.: C07C 233/60, C07C 231/12

(54) **Aminoacetal derivatives**
Aminoacetalderivate
Dérivés d'aminoacétal

(43) Date of publication of application: 27.05.1992
(73) Proprietor: AJINOMOTO CO., INC., Tokyo 104 (JP)
(72) Inventor: Kimura, Yasuhiro, c/o Ajinomoto Co., Inc., Chuo-ku, Tokyo (JP)
(74) Representative: Strehl Schübel-Hopf Groening & Partner

(56) References cited:
- EP-A- 0 201 693
- US-A- 4 508 594
- CHEMICAL ABSTRACTS, vol. 114, no. 21, 27th May 1991, page 769, abstract no.206636t, Columbus, Ohio, US; &JP-A-02 306 950 (AJINOMOT CO., INC.) 20-12-1990

## Description

### Field of the Invention

The present invention relates to novel aminoacetal derivatives which are useful intermediates for the synthesis of acrylamidoacetal derivatives useful as adhesives, coating agents or crosslinking agents, and to the synthesis of acrylamidoacetal derivatives via said compounds.

### Prior Art and its Problem

It is known that acrylamidoacetal derivatives are useful as adhesives, coating agents or crosslinking agents, and as diol-reactive crosslinking monomers. As processes for the synthesis of the compounds, there are known a direct process for synthesizing the compounds in one step by a condensation or substitution reaction between aminoacetals and acrylic acid derivatives and an indirect process in which the compounds are synthesized from aminoacetals and acrylic acid derivatives via intermediates. In the direct process, it is important to obtain the product from inexpensive raw materials in good yield; whereas in the indirect process, it is important to obtain the intermediates in good yield and to obtain the product from the intermediates in good yield. As a direct process, the following are known : 1) a process using acrylic acid cloride and aminoacetal (EP-A-13147, US-A-4215195, US-A-4247673, US-A-4346231, US-A-4421915, US-A-4438278); 2) dehydrative condensation between acrylic acid and aminoacetal (JP-A-59020262); 3) substitution reaction between acrylamide and aminoacetal; and 4) a process for ester-amide substitution between acrylic acid ester and aminoacetal (JP-A-59020262). As an indirect process, the following are known : 5) a process which comprises protecting the double bond in acrylic acid ester using an alcohol, then performing ester-amide substitution between aminoacetal and the protected acrylic acid ester and then removing the protecting alcohol (JP-A-59020262, JP-A-49069655). However, these processes involve the following problems. That is, the process 1) is not suitable for industrial production since acrylic acid chloride is extremely expensive. According to the processes 2) and 3), an acid catalyst is used in both processes so that the acetal is too unstable to obtain the product. In process 4), Michael's addition reaction is predominant so that the desired compound can hardly be obtained. In the case of 5), substitution of the ether bond occurs upon the ester-amide exchange reaction, in addition to the desired reaction so that it is difficult to isolate the product.

### [Problems to be solved by the Invention]

An object of the present invention is to provide novel aminoacetal derivatives for producing acrylamidoacetal derivatives useful as adhesives, coating agents or crosslinking agents in high yield. Another object is to provide a process for preparing the acrylamidoacetal derivatives via the aminoacetal derivatives.

### [Means for solving the Problems]

As a result of extensive investigations to obtain acrylamidoalkylaldehyde dialkylacetal derivatives (hereafter simply referred to as acrylamidoacetal derivatives) by the indirect process in high yield, the present inventors have found that aminoacetal derivatives can be excellent and novel intermediates for the synthesis of acrylamidoacetal derivatives and have developed a process for preparing the acrylamidoacetal derivatives via these intermediates. The present invention has thus been accomplished.

That is, the present invention relates to aminoacetal derivatives represented by the formula : 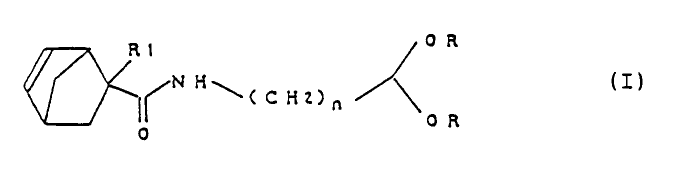
wherein n represents an integer of 1 to 10, R represents an alkyl group having 1 to 3 carbon atoms, and R₁ represents hydrogen or a methyl group; and to a process for preparing acrylamidoalkylaldehyde dialkylacetal derivatives represented by the formula (III) : 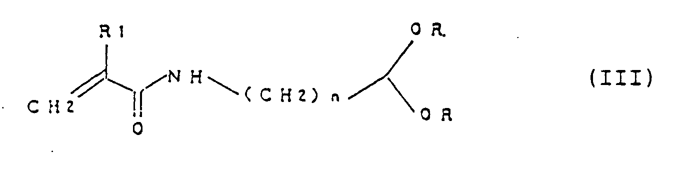
wherein n represents an integer of 1 to 10, R represents an alkyl group having 1 to 3 carbon atoms, and R₁ represents hydrogen or a methyl group, characterized in that an aminoacetal derivative represented by the formula (I) : 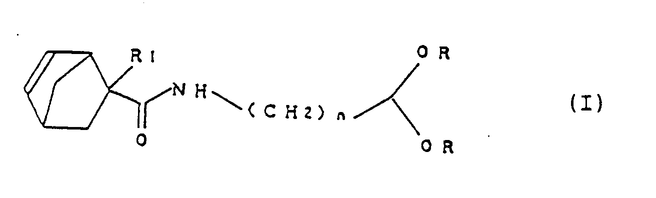
wherein n, R and R₁ are the same as in general formula (I) described above, is subjected to thermal decomposition.

The synthesis of the aminoacetal derivatives may be shown by the following reaction scheme : 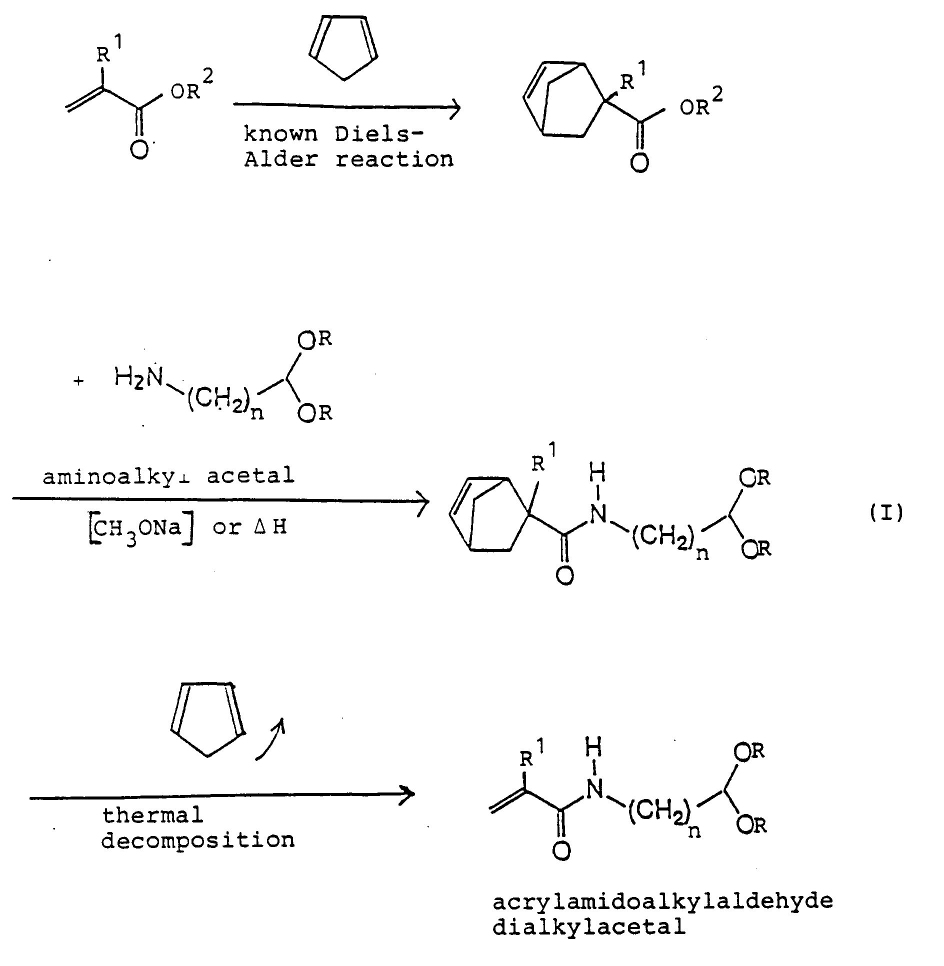
wherein R represents an alkyl group having 1 to 3 carbon atoms, R¹ represents hydrogen or a methyl group, R² represents an alkyl group having 1 to 4 carbon atoms, and n represents an integer of 1 to 10.

It may, for example, be performed as follows. Cyclopentadiene is added to methyl acrylate to obtain norbornenecarboxylic acid ester. Then the ester is reacted with the aminoacetal with heating to obtain the aminoacetal derivative in good yield. In the reaction between the aminoacetal and norbornenecarboxylic acid ester, by-products, which are noted in the reaction with an acrylic acid ester having a double bond protected with an alcohol, are not formed. Additionally to the reaction of the aminoacetal with norbornenecarboxylic acid ester by heating, aminolysis may also be carried out using as a catalyst an alkali metal alcoholate or a hydrogenated alkali metal, in order to accelerate the reaction. Examples of the acrylic acid ester which can be used herein are methyl, ethyl, propyl and butyl esters of acrylic or methacrylic acid. As an aminoacetal there may be used acetals derived from lower alcohols having 1 to 3 carbon atoms and from aldehydes such as aminomethylaldehyde, aminoethylaldehyde, aminopropylaldehyde, aminobutylaldehyde, aminopentylaldehyde, aminohexylaldehyde, aminoheptylaldehyde, aminooctylaldehyde, aminononylaldehyde and aminodecylaldehyde.

Furthermore, the acrylamidoacetal derivatives can be readily obtained also when acrylic acid derivatives are used. That is, cyclopentadiene is added to the acrylic acid derivative to obtain norbornenecarboxylic acid. Then, the carboxylic acid is reacted with thionyl chloride and the resulting acid chloride is reacted with the aminoacetal to give the acrylamidoacetal derivatives. Examples of the acrylic acid derivatives which can be used are acrylic acid and methacrylic acid. As aminoacetal there may be used acetals derived from lower alcohols having 1 to 3 carbon atoms and from aldehydes such as aminomethylaldehyde, aminoethylaldehyde, aminopropylaldehyde, aminobutylaldehyde, aminopentylaldehyde, aminohexylaldehyde, aminoheptylaldehyde, aminooctylaldehyde, aminononylaldehyde and aminodecylaldehyde. For the decomposition of the aminoacetal derivative by heating, in the case of using a catalyst such as alkali metal alkoxide, etc. in the reaction, it is required to neutralize the aminoacetal derivative with an equimolar amount of an acid and then perform thermal decomposition. When an excess of the acid is used, hydrolysis of the acetal is caused and such is not preferred. The thermal decomposition may be carried out using the method shown in, for example, Japanese Patent Application Laid-Open No. 49-66625. That is, either a method which comprises passing inert gas and steam of tie aminoacetal derivative through a reaction tower packed with a packing agent such as tubular Raschig rings, Raschig rings of Berl-saddle or McMahon type, etc., the material of which is made of ceramic, iron or carbon, at a temperature of 200°C to 800°C or a method which comprises performing thermal decomposition in an inert liquid such as paraffin, etc. and at the same time, distilling the product off. In order to prevent polymerization of the product, it is preferred that polymerization inhibitors such as hydroquinone derivatives, phenothiazines, catechols, etc. are added upon the thermal decomposition.

### Example 1

To 137 g of methyl acrylate were dropwise added 105 g of cylopentadiene. After stirring below 30°C overnight, 217 g of methyl norbornenecarboxylate were obtained, b.p. 72-75°C/7 Torr; yield, 90%.

After 17.5 g of 4-dimethoxybutylamine were added to 20 g of methyl norbornenecarboxylate, 1 g of 28% sodium methoxide was added to the mixture. The mixture was reacted at 120°C for 6 hours to give 35 g of the crude product. Further 0.13 ml of conc. sulfuric acid was added to the crude product. The mixture was distilled to give 30 g of 5-N-(4,4-dimethoxybutyl)carboxamido-2-norbornene; b.p. 160°C/1.2 Torr; yield, 90%.

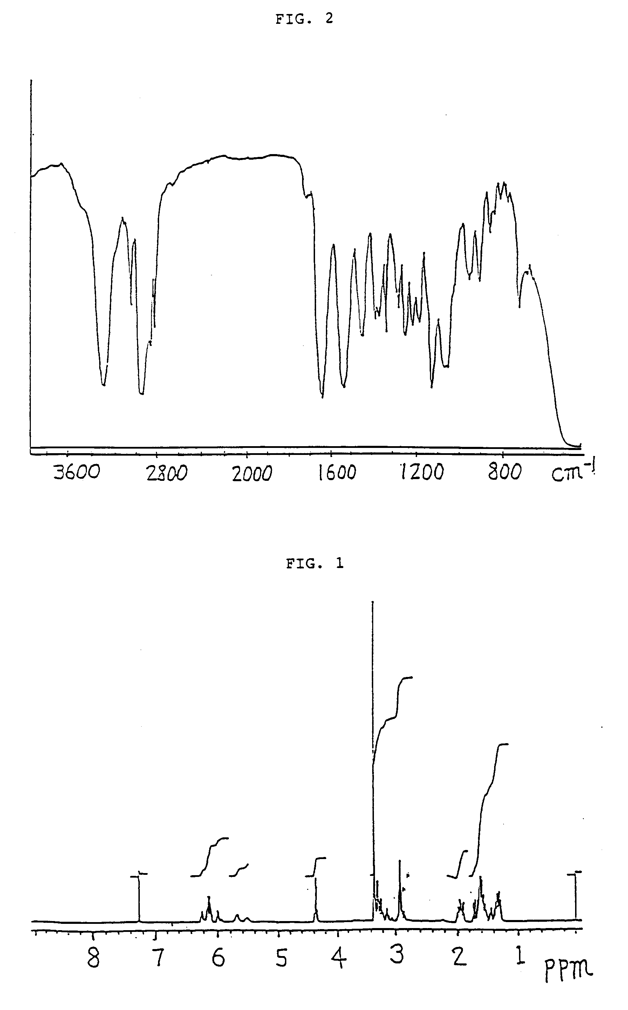

The product was confirmed by the NMR, IR and MASS spectra. The NMR and IR spectra are shown in Figs. 1 and 2.

Twenty five grams of 5-N-(4,4-dimethyloxybutyl)carboxyamide-2-norbornene were heated and vaporized under 0.3 Torr.

The vapor was thermally decomposed into acrylamide butylaldehyde dimethyl acetal (ABDA) and cyclopentadiene by passing it through a reaction tower (25x2.5 cm) heated to 550°C. Then, thermally decomposed vapor including ABDA was cooled to obtain ABDA and cyclopentadiene.

The crude ABDA compound was obtained by passing the vapor into a water-cooled apparatus, and then cyclopentadiene was also obtained by continuously setting cooling trap at -70°C for recycle-use.

Fourteen grams of crude ABDA were obtained under reduced pressure. The boiling point of the crude ABDA was 135°C at 0.77 Torr. The yield was 78 %.

### Effects of the Invention

The aminoacetal derivatives of the present invention can be prepared by the indirect process, without forming by-products at the stage of adding the aminoacetal and can be prepared from inexpensive raw materials.

### Brief Description of the Drawings

Figs. 1 and 2 show NMR and IR spectra of 5-N-(4,4-dimethoxybutyl)carboxamido-2-norbornene obtained according to the present invention.

## Claims

1. An aminoacetal derivative represented by the formula (I) : 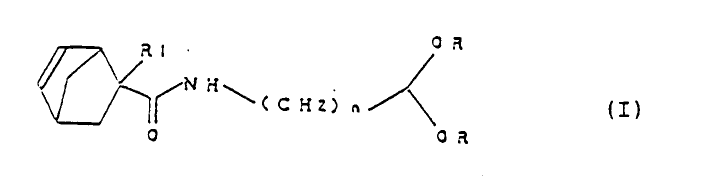 wherein n represents an integer of 1 to 10; R represents an alkyl group having 1 to 3 carbon atoms; and R₁ represents hydrogen or a methyl group.

2. An aminoacetal derivative according to claim 1, which is represented by the formula 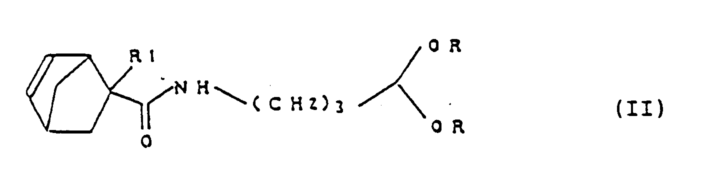 wherein R represents an alkyl group having 1 to 3 carbon atoms; and R₁ represents hydrogen or a methyl group.

3. A process for preparing an acrylamidoalkylaldehyde dialkylacetal derivative represented by the formula : 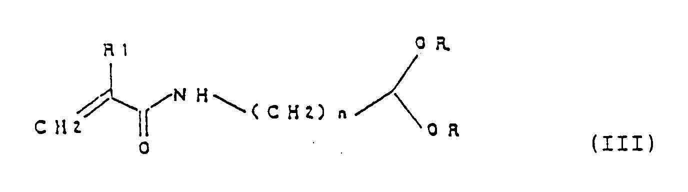 wherein n represents an integer of 1 to 10; R represents an alkyl group having 1 to 3 carbon atoms; and R₁ represents hydrogen or a methyl group, characterized in that an aminoacetal derivative represented by the general formula (I) 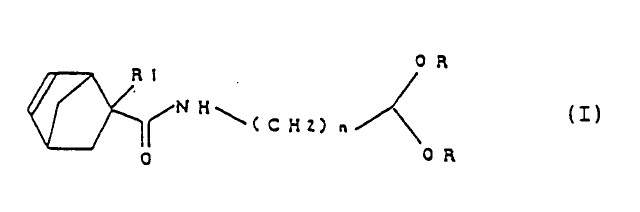 wherein n, R and R₁ are as defined in claim 1, is subjected to thermal decomposition.

## Patentansprüche

1. Aminoacetal-Derivat, dargestellt durch die Formel (I): 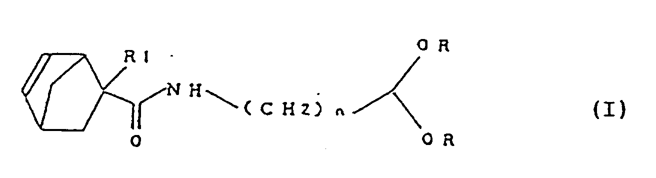 worin n eine ganze Zahl von 1 bis 10 bedeutet, R eine Alkylgruppe mit 1 bis 3 Kohlenstoffatomen darstellt und R₁ Wasserstoff oder eine Methylgruppe darstellt.

2. Aminoacetal-Derivat nach Anspruch 1, das durch die Formel 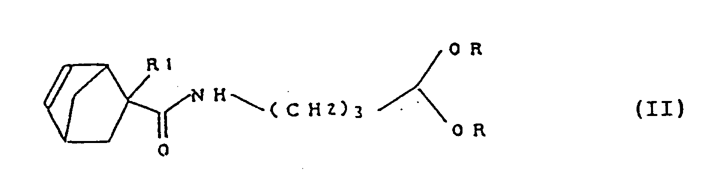 dargestellt ist,
worin R eine Alkylgruppe mit 1 bis 3 Kohlenstoffatomen bedeutet und R₁ Wasserstoff oder eine Methylgruppe darstellt.

3. Verfahren zur Herstellung eines Acrylamidoalkylaldehyddialkylacetal-Derivats, dargestellt durch die Formel 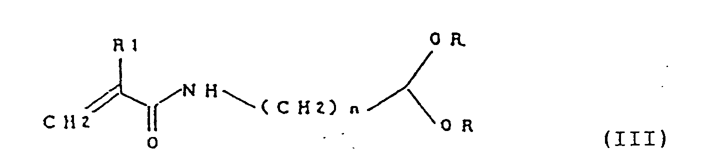 worin n eine ganze Zahl von 1 bis 10 bedeutet, R eine Alkylgruppe mit 1 bis 3 Kohlenstoffatomen bedeutet und R₁ Wasserstoff oder eine Methylgruppe darstellt, dadurch gekennzeichnet, daß ein Aminoacetal-Derivat, das durch die allgemeine Formel (I) dargestellt ist 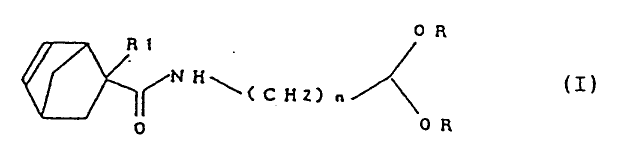 worin n, R und R₁ wie in Anspruch 1 definiert sind, der thermischen Zersetzung unterworfen wird.

## Revendications

1. Dérivé d'aminoacétal représenté par la formule (I) 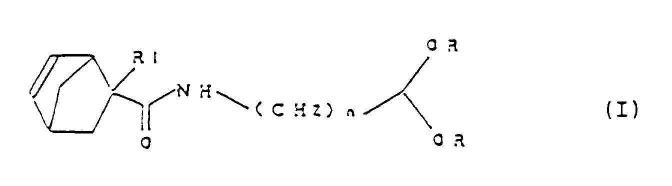 dans laquelle n représente un entier de 1 à 10 ; R représente un groupe alkyle en C₁-C₃ ; et R₁ représente un atome d'hydrogène ou un groupe méthyle.

2. Dérivé d'aminoacétal selon la revendication 1 qui est représenté par la formule 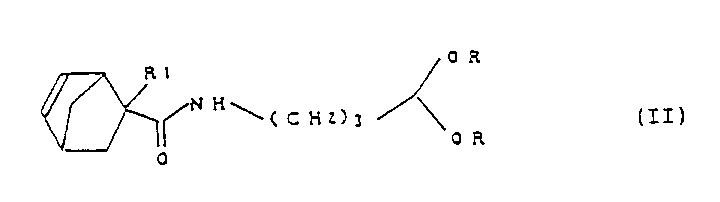 dans laquelle R représente un groupe alkyle en C₁-C₃ ; et R₁ représente un atome d'hydrogène ou un groupe méthyle.

3. Procédé pour préparer un dérivé dialkylacétal d'acrylamidoalkylaldéhyde représenté par la formule : 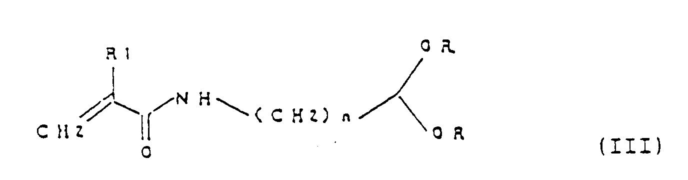 dans laquelle n représente un entier de 1 à 10; R représente un groupe alkyle en C₁-C₃ ; et R₁ représente un atome d'hydrogène ou un groupe méthyle, caractérisé en ce que l'on soumet à la décomposition thermique un dérivé d'aminoacétal représenté par la formule générale (I) : 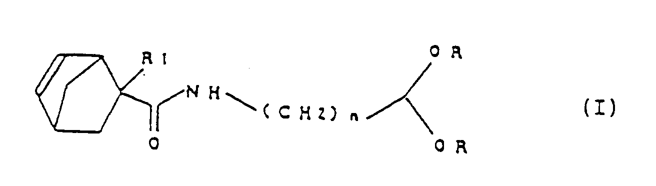 dans laquelle n, R et R₁ sont tels que définis dans la revendication 1.
